# EUROPEAN PATENT APPLICATION

(11) **EP 4 530 629 A1**
(43) Date of publication of application: **02.04.2025**
(21) Application number: 23306600.0
(22) Date of filing: 26.09.2023
(51) Int. Cl.: G01N 33/574, C12Q 1/6886

(54) **EX VIVO METHOD FOR PREDICTING THE RESPONSE TO AN IMMUNOTHERAPY TREATMENT OF HEPATOCELLULAR CARCINOMA IN A SUBJECT**

(71) Applicant: Université de Bordeaux, 33000 Bordeaux (FR); Institut National de la Santé et de la Recherche Médicale, 75013 Paris (FR); Centre Hospitalier Universitaire de Bordeaux, 33400 Talence (FR)
(72) Inventor: SALTEL, Frédéric, 33400 Talence (FR); RAYMOND, Anne-Aurélie, 33700 Merignac (FR); BLANC, Jean-Frédéric, 33800 Bordeaux (FR); Di TOMMASO, Sylvaine, 33600 Pessac (FR); DOURTHE, Cyril, 33700 Merignac (FR)
(74) Representative: Novagraaf Technologies

(57) **Abstract**

The present invention relates to an *ex vivo* method for predicting the response to an immunotherapy treatment of hepatocellular carcinoma in a subject, comprising the steps of:
1) determining the energy metabolism status of a liver tissue sample from the subject, said sample containing pathological tissue and optionally non-pathological tissue,
2) detecting the presence or the absence of an energetic shift consisting of a reduction of mitochondrial respiration, in pathological tissue compared to non-pathological tissue from the liver tissue sample of the subject or from a standard non-pathological liver sample,
Wherein:
- If mitochondrial respiration is reduced in pathological tissue, the subject is unlikely to have a response to the immunotherapy treatment,
- If mitochondrial respiration is not reduced in pathological tissue, the subject is likely to have a response to the immunotherapy treatment.

## Description

### Technical field

The present invention refers to an ex *vivo* method for predicting the response to an immunotherapy treatment of hepatocellular carcinoma in a subject.

Therefore, the present invention has utility in medical field, and more particularly in diagnosis, prognosis and treatment response prediction fields.

In the description below, the references into brackets ([ ]) refer to the listing of references situated at the end of the text.

### Background of the Invention

The therapeutic arsenal against cancer is expanding day-by-day. However, it is essential for oncologists to identify which treatment will actually be beneficial to each patient in order to strive towards the goal of precision medicine. Some targeted therapies already benefit from companion tests. However, detecting mutation of the targeted oncogene or labeling the immunotherapy target by immunohistochemistry does not guarantee an objective response. Today, some cancers, including liver cancer, cannot benefit from any current tools for treatment choice orientation.

Liver cancer is a major health problem and is the second leading cause of cancer death worldwide, with more than 850,000 new cases each year. There has been an increase in the number of cases over the past 20 years in Western countries. Hepatocellular carcinoma (HCC) is the most common among primary liver cancers. HCC frequently develops on cirrhosis but rarely in healthy liver. While cirrhosis surveillance programs can detect a large proportion of early-stage HCC, for which curative treatment is available (surgical resection, percutaneous destruction, liver transplantation), 50-70% of HCCs are indeed diagnosed late. Advanced HCC, with extremely poor prognosis, can only be treated with regional (arterial chemoembolization) or palliative systemic therapies.

The role of liver biopsy in the diagnosis and management of HCC has always been questioned. So far, the use of liver biopsies in the management of HCC has been limited due to the risk of complications, such as tumor seeding and bleeding, as well as the availability of effective non-invasive diagnosis by imaging. However, it is now widely accepted that these risks are infrequent, manageable, and do not affect the disease course; these reasons should therefore not be considered for avoiding liver biopsy. Conversely, the risk of insufficient sampling is also highlighted by many studies. This is mainly due to the fact that genetic mutations or transcriptomic profiles associated with HCC have not shown any predictive value for currently proposed treatments. Indeed, liver tumor biopsies are undoubtedly underrated and underused, especially given that fixed biopsies contain valuable information that could reverse the current risk/benefit ratio.

Sorafenib was approved in most countries and until recently as the standard first-line therapy for advanced HCC. Sorafenib originally demonstrated survival benefits despite a very low number of responders and significant side effects, but several phase III clinical trials have subsequently failed to demonstrate a superior survival benefit in advanced HCC. The arrival on the market of new drugs with proven efficacy was thus a revolution in the palliative care of HCC. A different phase III study has shown the non-inferiority of lenvatinib compared to sorafenib in overall survival of patients with advanced HCC with equivalent safety and tolerability profiles, thus justifying its use in first-line treatment. According to clinical recommendations, patients can also receive second-line treatments including cabozantinib, ramucirumab, nivolumab, or pembrolizumab. In addition, after initial promising results of immunotherapy treatments (nivolumab, pembrolizumab), the combination of atezolizumab (anti-programmed death-ligand 1 (PDL1) antibody) and bevacizumab (anti-vascular endothelial growth factor (VEGF) antibody) has recently shown a dramatic benefit in first line compared to standard care with sorafenib. This combination has indeed become the new standard first-line treatment (Gordan JD et al. ([1])). This strengthened therapeutic arsenal represents a considerable improvement having already demonstrated better-preserved liver function and an increase in median overall survival from six months to over 26 months in patients with advanced HCC. However, the therapeutic strategy guiding the choices of first-line treatment and/or therapeutic sequences are not defined. Consequently, it is crucial to predict response to these different treatments to improve their efficiency.

Laser capture and mass spectrometry (MS) analysis have previously been combined to thoroughly analyze the proteomic profiles of tumors (Henriet E et al. ([2]), Dourthe C et al. ([3])). This methodology, compatible with formalin-fixed and paraffin-embedded tissues (FFPET) as well as very small quantities of material (diagnostic biopsies), has already enabled to identify a biomarker; ASS1, now used for the diagnosis of sonic hedgehog hepatocellular adenomas associated with a high bleeding risk (Henriet E et al. ([2]), Sala M et al. ([4])). Moreover, it is now possible to perform proteomic profile matching in clinical routine using a machine-learning tool recently developed. Indeed, comparing the proteomic profiles of tumors with those of a reference database can make a diagnosis or predict a malignant transformation (Dourthe C et al. ([3])).

However, there is no precision medicine tool to enable the prognosis of each cancer. As an example, apart from contraindications to treatment, there is no biological rationale that can be used to guide the choice of treatment in hepatocellular carcinoma (HCC). The atezolizumab/bevacizumab immunotherapy combination is given as a first-line priority, as it has the best objective response rate (20-30%). However, in the absence of prognostic tool in the treatment of advanced hepatocellular carcinoma, in the event of failure of a first-line treatment, only 25% of patients have time to try a second line of treatment.

Thus, a need exists of new tools for predicting the response to treatment of hepatocellular carcinoma. The present invention fulfills these and other needs.

### Description of the invention

After extensive research work, the Applicant has surprisingly shown that the enrichment of certain biological pathways, especially energy metabolism, is a very discriminant and robust tool for predicting the response of HCC to immunotherapy, notably atezolizumab/Bevacizumab immunotherapy.

Therefore, the invention is based on using tumor energy metabolism status as a new tool to predict the response to immunotherapy in HCC.

One of the major advantages of the invention is that it makes it possible to direct patients towards another potentially more suitable treatment, such as, for example, repositioning multikinase inhibitors (lenvatinib or sorafenib) if the tumor presents an objective response profile.

Another advantage of the invention is that it can be performed using diagnostic biopsies made before treatment. Therefore, only small amounts of tissue are needed for this analysis, thus not depleting the samples and it can be reused.

Another impressive advantage of the process is that the complete analysis can be achieved within a week and so it remains compatible with the deadlines imposed by clinical practice. Moreover, the proteomic data, when generated, remains a resource that can be re-exploited for further applications (e.g. prognosis, prediction of the response of other treatments, search for new pharmacological targets).

In addition, the Applicant developed a response prediction approach, notably proteomic profiling-based, for implementing the predicting process of the invention. Another advantage is that proteomic profiling combined with a predictive signature for response to treatment enable the most appropriate treatment to be prescribed directly to each patient without any loss of chance, and thus increase life expectancy in HCC.

Accordingly, in a first aspect, the present invention provides an ex *vivo* method for predicting the response to an immunotherapy treatment of hepatocellular carcinoma in a subject, comprising the steps of:
1) determining the energy metabolism status of a liver tissue sample from the subject, said sample containing pathological tissue and optionally non-pathological tissue,
2) detecting the presence or the absence of an energetic shift consisting of a reduction of mitochondrial respiration, in pathological tissue compared to non-pathological tissue from the liver tissue sample of the subject or from a standard non-pathological liver sample ,

Wherein:
- If mitochondrial respiration is reduced in pathological tissue, the subject is unlikely to have a response to the immunotherapy treatment,
- If mitochondrial respiration is not reduced in pathological tissue, the subject is likely to have a response to the immunotherapy treatment.

"Hepatocellular carcinoma" or HCC refers herein to any HCC, regardless of its stage of development. As an example, it may be an early-stage HCC, a mid-stage HCC or an advanced HCC, and a resectable or a non resectable HCC. The HCC may already have been treated with a first line therapy, or not.

As used herein, a "subject" is a patient, animal, mammal, or human, suffering from HCC.

According to the invention, the immunotherapy treatment of HCC may be any treatment of HCC by activating the immune system of the subject, which is already known in the art. It may be a treatment already approved in at least one country by a drug regulatory authority, or not approved and undergoing testing. The treatment may be a single molecule or a combination of at least two molecules. Immunotherapy treatment may be treatment with monoclonal antibodies. Monoclonal antibodies for use in immunotherapy may be naked, i.e., non-conjugated, or conjugated, i.e., have a chemotherapy drug or radioactive particle attached to them. Further, monoclonal antibodies for cancer immunotherapy can be bispecific, i.e., designed to recognize and bind to two different proteins. For example, the immunotherapy treatment may be chosen among anti-PDL1 monotherapy such as nivolumab, atezolizumab and bevacizumab combination therapy, anti-PDL1 and anti-VEGF combination therapy, anti-PD1 and CTLA4 combination therapy, PDL1 and CTLA4 combination therapy, anti-VEGFR2 therapy such as ramucirumab therapy, and anti-PD1 therapy such as pembrolizumab therapy.

The liver tissue sample may be a physical tissue sample or an image of the liver tissue from medical imaging. Therefore, the liver tissue sample may be any sample obtainable by known techniques, as example by at least one technique selected among macrodissection, laser microdissection of a formalin-fixed paraffin-embedded or frozen tissue, biopsies, notably fresh biopsy, and positon emission tomography. Any surface area of tissue sample may be used, as long as it contains pathological tissue and optionally non-pathological tissue.

"Pathological tissue" refers herein to liver tissue having HCC cancerous cells.

"Non-pathological tissue" refers herein to liver tissue that does not have HCC cancerous cells.

Advantageously, the liver tissue sample from the subject contains pathological and non-pathological tissue.

"Energy metabolism" refers herein to all or part of the biological pathways involved in the process of generating energy (i.e. ATP) from nutrients in the liver tissue sample of a subject. Energy metabolism involves generally the following pathways: mitochondrial respiration pathways, including oxidative phosphorylation, TCA cycle and fatty acid beta oxidation. The status of the energy metabolism refers to the level of activation of all or part of these metabolic pathways. Therefore, the energy metabolism status to be determined in the method of the invention may comprise determining mitochondrial respiration pathways, including oxidative phosphorylation, TCA cycle and fatty acid beta oxidation, or determining at least one pathway chosen among oxidative phosphorylation pathway, TCA cycle pathway and fatty acid oxidation pathway. Advantageously, the energy metabolism status comprises the biological pathways oxidative phosphorylation and TCA cycle.

In step 1) of the method, determining the energy metabolism status may be determined by any method known by the skilled person allowing to measure metabolic activity in a tissue. It may be for example at least one method selected among proteomic profiling matching, immunohistochemistry, western blot, global or targeted mass spectrometry analysis, reverse transcriptase PCR and positon emission tomography (PET). In this regard, proteomic profiling matching, immunohistochemistry, western blot, global or targeted mass spectrometry analysis and reverse transcriptase PCR may be used when the liver tissue sample is a physical tissue sample. PET may be used when the liver tissue is an image of the liver tissue from medical imaging. It may be a direct measure of the expression level of metabolic enzymes, or an indirect measure of the expression level of metabolic enzymes via the measure of the transcripts level, or a measurement of mitochondrial mass, for example using histological techniques such as immunohistochemistry or immunofluorescence, or medical imaging to measure the status of energy metabolism using a labelled tracer.

In a preferred embodiment of the invention, the energy metabolism status may be determined by investigating expression of proteins involved in the energy metabolism. For example, determining the energy metabolism status may be carried out by determining abundance of proteins involved in the energy metabolism of said liver tissue sample from the subject. It may be performed directly, i.e. by quantifying the proteins in the tissue, or indirectly, i.e. by measuring mRNA of said proteins.

Determining the abundance of proteins may be carried out by any method known in the state of the art. It may be a relative protein abundance, or a quantified protein abundance.

In case of determining relative protein abundance, any method known in the state of the art may be used. For example, it may be a mass spectrometry based analysis of the relative protein abundance of proteins involved in the energy metabolism between pathological and non pathological tissue. More precisely, it may be the relative abundance of each protein of the non-pathological tissue over the corresponding protein in the pathological tissue, or vice versa.

In case of quantifying protein abundance, it may be carried out by any method known by the skilled person, for example a targeted quantification technique. It may be for example a method of liquid chromatography coupled with tandem mass spectrometry (LC-MS/MS), in discovery mode or with a targeted approach (eg. Multiple Reaction Monitoring (MRM), Parallel Reaction Monitoring (PRM)), in Data Dependant Acquisition (DDA) or Data-independent Acquisition (DIA).

Determination of the energy metaboslim status is realized in the pathological tissue of the liver tissue sample of the subject. If the liver tissue is also intended to contain non-pathological tissue, the determination may also be realized in the non-pathological tissue. Advantageously, in this case, detecting the energetic shift is realized by comparing pathological tissue and non-pathological tissue of the subject.

In another embodiment, the comparison may be made between the pathological tissue of the subject and a standard non-pathological tissue of liver sample, used as a reference, for example an extract of normal liver tissue from one patient or a mixture of several patients. Alternatively, the comparison may be made with a reference, pre-determined, energy metabolism status, which can be obtain from a non-pathological tissue liver sample from a third party, or from several non-pathological tissue liver samples in order to be statistically representative.

"Reduction of mitochondrial respiration" refers herein to a decrease of the activation of the biological pathways involved in the mitochondrial respiration, notably in favor of the glycolysis pathway. Without intending to be bound by any theory, this corresponds to an energy shift, also called Warburg effect, in which pyruvate is converted to lactate at the expense of mitochondrial activity via glycolysis. It is an hypothesis that in the context of energetic shift, tumor hypoxia, low pH, suppressive metabolites and low nutrient availability can all prevent T cells from acquiring optimal effector functions, even if the antibody target is well expressed, thereby explaining non responding to immunotherapy. The reduction may be any statistically significant reduction. Statistical analysis may be realized by any method known for similar purpose in the state of the art, depending on the method used and the dataset to be tested. It may be for example a Wilcoxon-Mann-Whitney U-test or a t-test. The skilled person is able to choose one or more of statistical analysis regarding his knowledge of this technical field.

In an embodiment of the invention, the energy metabolism status refers to determining the mitochondrial respiration pathways as defined above, and, in addition, determining glycolysis pathway, which should not be modified, i.e. should be stable, or may be increased in case of Warburg-type metabolic shift.

"Likely to have a response" to immunotherapy treatment refers herein to a statistically significant level of tumor regression and/or decrease in alpha-fetoprotein (AFP) levels. It may be for example a tumor regression according to the RECIST criteria, which considers that tumor regression to be significant at 30% tumor mass and 50% AFP. Alternatively, it may be for example a tumor regression and/or decrease in AFP levels, in the first two months after treatment initiation, of at least 5%, or at least 10%, or at least 20%, or at least 30%, or at least 40%, or at least 50%, or at least 60%, or at least 70%, or at least 80%, or at least 90%, or even 100%, or any number or range in between, compared with the value without immunotherapy treatment.

"Unlikely to have a response" to immunotherapy treatment refers herein to the other cases. It may be a tumor progression according to the RECIST criteria, which consider that a tumor progression is significant at 20% tumor progression. Alternatively, it may be a tumor progression to a similar extent to a case without treatment, for example a tumor progression of at least 5%, or at least 10%, or at least 20%, or at least 30%, or at least 40%, or more, within two months.

In another aspect, the present invention provides a method for choosing a treatment for a patient with HCC for an immunotherapy treatment, comprising the steps of carrying out the ex vivo prediction method as defined above, and selecting the patient for whom an energetic shift is not detected.

In another aspect, the present invention provides a method of treating a patient with HCC, comprising the steps of:
(i) selecting the patient for treatment for whom an energetic shift is not detected, and
(ii) administering to the patient an effective amount of immunotherapy treatment.

This invention is further illustrated by the following examples with regard to the annexed drawings that should not be construed as limiting.

### Brief description of the figures

- Figure 1: represents principal component analysis of the proteomic profiles showing deregulated protein expressions between tumoral vs non-tumoral (T/NT) tissues in patients with an objective response (ORuAB n=6) or with progression (PuAB n=12) under immunotherapy atezolizumab/bevacizumab. The dataset was reduced to a signature of 113 proteins with significantly different expression between patients with ORuAB (grey) and PuAB (black).
- Figure 2: represents Bootstrap analysis of the signature differentiating PuAB and ORuAB patients. 54/113 (48%) proteins were significant in over 80% of resampling configurations.
- Figure 3: represents gene Set Enrichment Analysis obtained from Gene Ontology database (cellular component) using the 113 proteins that were significantly differentially expressed between patients with objective response (ORuAB) versus progression (PuAB) under atezolizumab/bevacizumab combination.
- Figure 4: represents gene Set Enrichment Analysis obtained from Ingenuity Pathways database (IPA Qiagen, canonical pathways) using the 113 proteins that were significantly differentially expressed between patients with objective response (ORuAB) versus progression (PuAB) under atezolizumab/bevacizumab combination.
- Figure 5: represents ratio of Tumor vs Non-Tumor (T/NT) expression of proteins involved oxidative phosphorylation for patients who progressed (PuAB) or had an objective response (ORuAB) to atezolizumab/bevacizumab immunotherapy (ratios expressed in log).
- Figure 6: represents ratio of Tumor vs Non-Tumor (T/NT) expression of proteins involved in TCA cycle for patients who progressed (PuAB) or had an objective response (ORuAB) to atezolizumab/bevacizumab immunotherapy (ratios expressed in log).
- Figure 7: represents ratio of Tumor vs Non-Tumor (T/NT) expression of proteins involved in glycolysis for patients who progressed (PuAB) or had an objective response (ORuAB) to atezolizumab/bevacizumab immunotherapy (ratios expressed in log).

### Examples

### Example 1: Predicting response to immunotherapy in hepatocellular carcinoma using tumor energy metabolism status

### Materials and Methods

### Patients

Patients with advanced HCC treated with atezolizumab/bevacizumab were selected and divided into two groups according to their response to treatment. The response was determined according to radiological tumor assessment and/or alpha-fetoprotein (AFP) levels assessed in the first two months after treatment initiation. In this example, an objective response corresponded to a >30% tumor regression and/or a >50% decrease in AFP levels. A >20% tumor progression corresponded to no response. For study inclusion and prior to the start of treatment, each patient had a biopsy taken with sufficient tumoral and non-tumoral tissue (>1mm²) for subsequent proteomic analysis by MS.

### Laser capture

Liver tissue sections from each patient were stained with hematoxylin-eosin-saffran for histopathological examination and determination of the areas of interest for dissection. Tumoral (T) and non-tumoral (NT) liver of the same surface area and homogeneous tissue composition (absence of fibrosis) were then selected under anatomopathologist supervision. Tumoral and non-tumoral tissue (1mm²), chosen beforehand from adjacent serial hematoxylin-eosin-stained sections, were micro-dissected from 5µm-thick FFPE sections using a PALM type 4 (Zeiss) laser micro-dissector. The same tissue area was cut into serial sections and treated in the same way to form three technical replicates.

### Sample preparation for proteomic analysis

Fixation reversion and protein extraction from tissue sections, *in gel* digestion of proteins, peptide extraction from gel, and peptide sample preparation for LC-MS/MS analysis were performed as previously described (Sala M et al. ([4])).

### Mass spectrometry analysis

Online nano LC-MS/MS analyzes were performed using an Ultimate 3000 RSLC Nano-UPHLC system (Thermo Scientific, USA) coupled to a Nanospray Q-Exactive hybrid quadrupole-Orbitrap mass spectrometer or a Nanospray Orbitrap Fusion^{™} Lumos^{™} Tribrid^{™} Mass Spectrometer (Thermo Scientific, USA) as previously described (Henriet E et al. ([2]), Dourthe C et al. ([3]), Sala M et al. ([4])).

### Database search, MS result processing and quantification

For protein identification, the Mascot 2.5 algorithm in the Proteome Discoverer 2.5 Software (Thermo Fisher Scientific Inc.) was used in batch mode by searching against the UniProt Homo sapiens database (71 793 entries, Reference Proteome Set, release date: December, 2017) from website: http://www.uniprot.org/. Two missed enzyme cleavages were permitted. Mass tolerances in MS and MS/MS were set to 10 ppm and 0.02 Da. Oxidation of methionine and acetylation of lysine were searched as dynamic modifications. Cysteine carbamidomethylation was searched as a fixed modification. Raw LC-MS/MS data were imported in Proline Web for feature detection, alignment, and quantification. Protein identification was only accepted when at least 2 specific peptides had a pretty rank=1 and a protein false discovery rate value <1.0% calculated using the "decoy" option in Mascot. Label-free quantification of proteomic data using MS1-extracted ion chromatograms was carried out using the parameters previously indicated (Henriet E et al. ([2]), Dourthe C et al. ([3]), Sala M et al. ([4])). Normalizations were made to median ratios. The inference of missing values was applied to the 5% background noise.

### Bioinformatics analysis

Differential protein expression between tumoral and non-tumoral tissue samples from each patient was analyzed by computing log-ratios of the median of normalized protein abundances for each pair of tumoral/non-tumoral tissue samples.

We performed the Welch's t-test (not assuming equal population variance) to reveal potential biomarkers distinguishing between objective response (ORuAB) and progression under sorafenib (PuAB). We set the significance threshold for the adjusted p-value to 0.05, resulting in a predictive signature of 113 proteins.

Using this dataset, we performed principal component analysis using FactoMineR and Factoextra available in R. The bootstrap analysis was carried out using R's database packages (base and stats packages). In order to find pathways which were significantly deregulated between ORuAB and PuAB we performed Gene Set Enrichment Analysis on the 113-protein signature against Gene Ontology (GO) database (cellular components) and the Ingenuity Pathways (IPA, Qiagen) database (canonical pathways).

### Results

Using tissue proteomic profiling methodology, we first compared relative protein expression levels between tumoral and non-tumoral tissues for each patient; thus generating the proteomic profile of HCC (Dourthe C et al. ([3])). The analysis was performed on FFPE liver biopsies taken prior to starting treatment with atezolizumab/bevacizumab combination. The analysis strategy was designed to limit bias due to unavoidable variabilities. On one hand, three technical replicates were designed to limit technical variability of quantification. Also and above all, comparing the tumoral versus non-tumoral protein expression ratio for each patient reduced background noise caused by inter-individual variability, thus acquiring greater statistical power. This methodology allowed us to obtain robust data (Henriet E et al. ([2]), Dourthe C et al. ([3])) on even a relatively small cohort.

### Distinct proteomic profiles for tumors depending on response to atezolizumab/bevacizumab combination

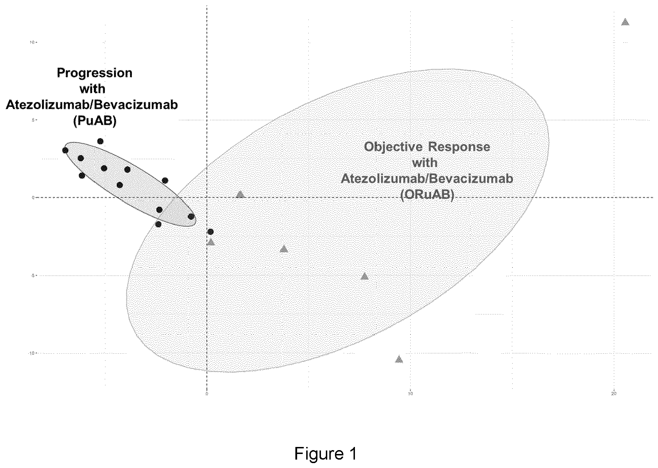

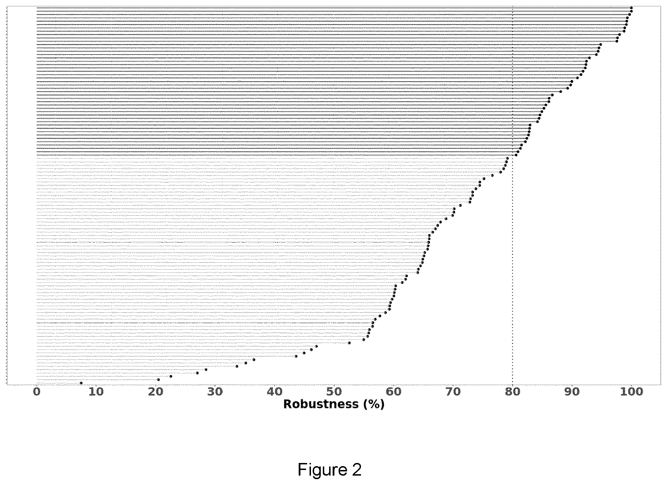

To identify a proteomic profile capable of predicting ORuAB and PuAB, we applied the adjusted Wilcoxon t-test comparing the TINT ratios from patients in ORuS and PuS groups. We set the significance threshold for the adjusted p-value to 0.05, generating a signature of 113 proteins. Patients in the ORuAB versus PuAB groups were well distinct in principal component analyzes of differential protein expression profiles (Figure 1). We tested the robustness of this signature using a bootstrap method of resampling the samples 10,000 times. We indeed demonstrated that 54/113 (48%) proteins were >80% significantly accurate (Figure 2). This means that over half of the identified response prediction signature was highly recurrent. However, this does not mean that the remaining proteins have no predictive value, more so that their deregulated expression was more variable within the ORuAB and PuAB groups.

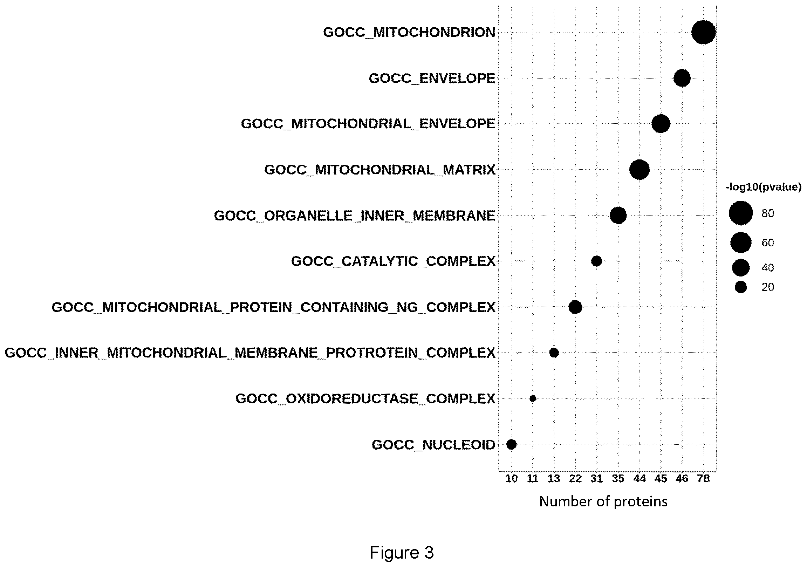

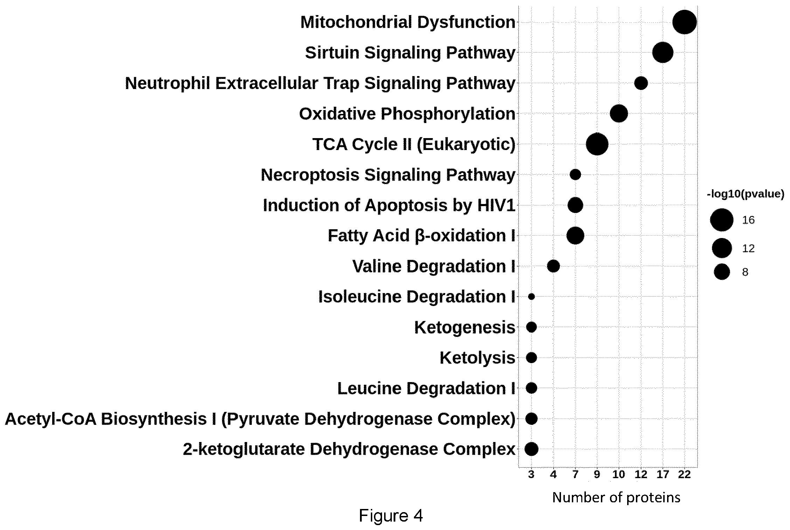

From a functional perspective, at least 70% of the proteins in the signature were associated with a mitochondrial function, as shown by the Gene Set Enrichment Analysis carried out against the Gene Ontology database (GO, cellular component) (Figure 3). Consistently, the biological pathways most enriched in this signature were associated with mitochondrial energy metabolism functions (oxidative phosphorylation, TCA cycle, fatty acid beta oxidation), as shown by the Gene Set Enrichment Analysis carried out against the Ingenuity Pathways database (IPA, Qiagen, canonical pathways) (Figure 4).

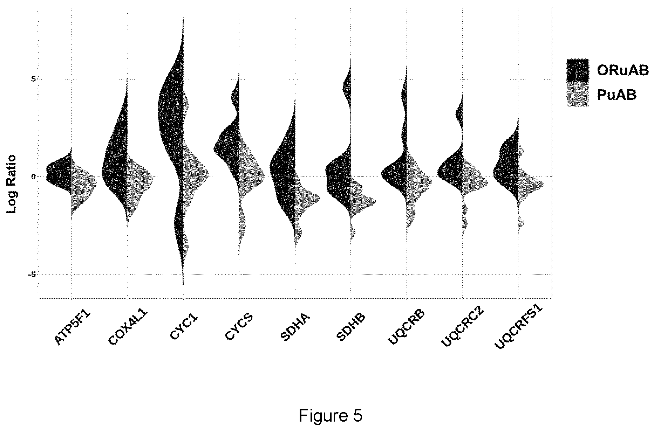

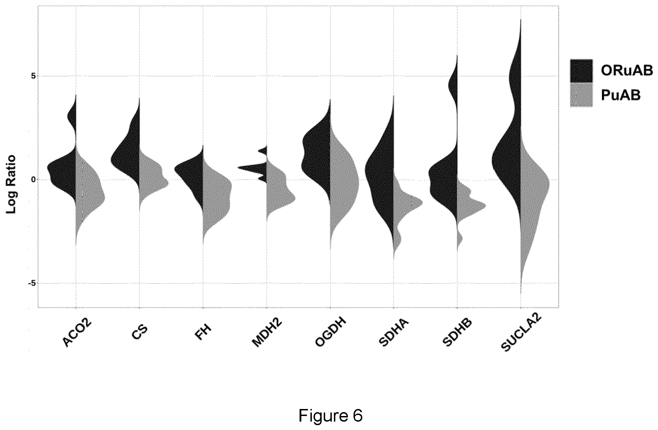

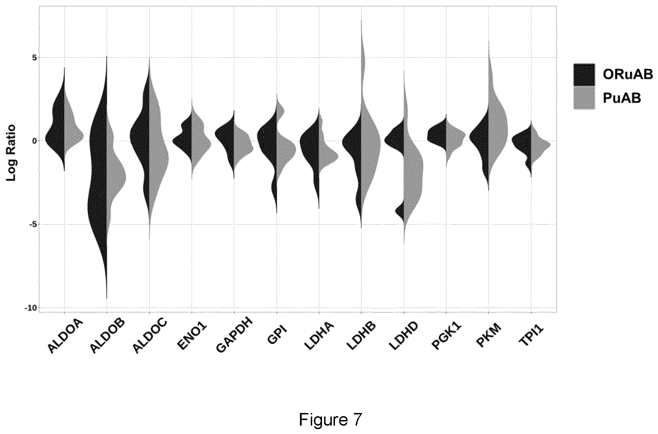

Focusing on the TINT ratios of each protein, we found that patients who progressed (PuAB) on immunotherapy had a significant and general decrease of the expression of mitochondrial respiration proteins in the tumor (oxidative phosphorylation (Figure 5), TCA cycle (Figure 6)), whereas patients who had an objective response (ORuAB) had TINT expression ratios >1 and sometimes significantly overexpressed in the tumor. However, the glycolysis proteins expression was not significantly altered in PuAB or ORuAB nor did it differ between the two groups of patients (Figure 7).

### Discussion

The lack of predictive markers for response to treatments in advanced HCC still remains a big challenge despite the many studies already performed. These predictive markers could change the way patients are treated, especially due to availability of new treatments. To date, three different therapeutic options are available in first line for advanced HCC: sorafenib, lenvatinib, and more recently the atezoliumab/bevacizumab combination. It is obvious that patients will respond differently to these treatments. Other points to consider are the adverse effects of these drugs and their substantial healthcare costs. Consequently, we need to strive for precision medicine to determine the optimal treatment for each patient. All the required information for this response prediction strategy is actually available in fixed diagnostic biopsies.

Combining laser capture and MS allowed to access deregulated protein expression profiles from fixed biological tissue samples available in routine clinical practice. Indeed, one major advantage of the approach according to the invention is the broad applicability given needle biopsies are used in routine care, and the potential of this approach in predicting responses to treatment in HCC.

The results we obtained have shown a decrease in oxidative metabolism in tumors that did not respond to immunotherapy treatment, probably in favor of an increase in glycolytic flux without modification of expression of glycolysis proteins.

To supply the cell with energy, glucose enters the cell via transporters. It is then metabolized to pyruvate by a series of enzymatic reactions.

In a context of effective oxidative phosphorylation, pyruvate enters the mitochondria where it is converted to acetyl-CoA, which is degraded in the Krebs cycle to produce energy via the electron transport chain of the inner mitochondrial membrane.

In the event of an energy shift (called Warburg effect), pyruvate is converted to lactate at the expense of mitochondrial activity, and is expelled from the cell by monocarboxylate transporters (MCTs). The lactate secreted then contributes to extracellular acidification (DePeaux K et al. ([5])).

As already mentioned in the literature (21), a high rate of glycolysis can lead to local depletion of oxygen and glucose levels (hypoxic regions with high levels of lactate).

In addition, increased tumor proliferation is often associated with deregulation of the vasculature (damaged and leaky vessels), which reduces blood flow. Immunosuppression is established and the cells cannot leave the tumor.

Anti-cancer immunotherapeutic strategies based on checkpoint inhibition require sustained effector T cell responses to induce durable clinical responses. T cells need to enter and thrive in the tumor microenvironment. However, in the context of metabolic shift, tumor hypoxia, low pH, suppressive metabolites and low nutrient availability can all prevent T cells from acquiring optimal effector functions, even if the antibody target is well expressed. This is probably what happened in the HCC of the patients we have analyzed who have progressed under immunotherapy. The difference in energetic metabolic status is so discriminating between the two groups of patients with HCC who have responded or not to immunotherapy that it allows using metabolic shift detection alone to guide treatment.

### Reference List

1. Gordan JD, Kennedy EB, Abou-Alfa GK, Beg MS, Brower ST, Gade TP, et al. Systemic Therapy for Advanced Hepatocellular Carcinoma: ASCO Guideline. JCO. 2020;38:4317-4345.
2. Henriet E, Abou Hammoud A, Dupuy J-W, Dartigues B, Ezzoukry Z, Dugot-Senant N, et al. Argininosuccinate synthase 1 (ASS1): A marker of unclassified hepatocellular adenoma and high bleeding risk. Hepatology. 2017;66:2016-2028.
3. Dourthe C, Julien C, Di Tommaso S, Dupuy J, Dugot-Senant N, Brochard A, et al. Proteomic profiling of hepatocellular adenomas paves the way to new diagnostic and prognostic approaches. Hepatology. 2021;hep.31826.
4. Sala M, Gonzales D, Leste-Lasserre T, Dugot-Senant N, Paradis V, Di Tommaso S, et al. ASS1 Overexpression: A Hallmark of Sonic Hedgehog Hepatocellular Adenomas; Recommendations for Clinical Practice. Hepatol Commun. 2020;4:809-824.
5. DePeaux K, Delgoffe G. Metabolic barriers to cancer immunotherapy. Nat Rev Immunol. 2021 Dec;21(12):785-797.

## Claims

1. An ex *vivo* method for predicting the response to an immunotherapy treatment of hepatocellular carcinoma in a subject, comprising the steps of:
1) determining the energy metabolism status of a liver tissue sample from the subject, said sample containing pathological tissue and optionally non-pathological tissue,
2) detecting the presence or the absence of an energetic shift consisting of a reduction of mitochondrial respiration, in pathological tissue compared to non-pathological tissue from the liver tissue sample of the subject or from a standard non-pathological liver sample,
wherein:
- If mitochondrial respiration is reduced in pathological tissue, the subject is unlikely to have a response to the immunotherapy treatment,
- If mitochondrial respiration is not reduced in pathological tissue, the subject is likely to have a response to the immunotherapy treatment.

2. An ex vivo method according to claim 1, wherein the energy metabolism status comprises at least one pathway chosen among mitochondrial respiration pathways, oxidative phosphorylation pathway, TCA cycle pathway and fatty acid oxidation pathway.

3. An ex *vivo* method according to anyone of the preceding claims, wherein the energy metabolism status is determined by at least one method selected among proteomic profiling matching, immunohistochemistry, western blot, global or targeted mass spectrometry analysis, reverse transcriptase PCR and positon emission tomography.

4. An ex *vivo* method according to anyone of the preceding claims, wherein determining the energy metabolism status is carried out by determining abundance of proteins involved in the energy metabolism of said liver tissue sample from the subject, either directly or indirectly, for example by measuring mRNA of said proteins or by measuring mitochondrial mass.

5. An ex *vivo* method according to claim 4, wherein determining the abundance of proteins is carried out by label free proteomic analysis of the relative protein abundance of proteins involved in the energy metabolism between pathological and non pathological tissue.

6. An ex *vivo* method according to claim 5, wherein the protein abundance is the relative abundance of each protein of the non-pathological tissue over the corresponding protein in the pathological tissue.

7. An ex *vivo* method according to claim 4, wherein determining abundance of proteins is carried out by a targeted quantification technique.

8. An ex *vivo* method according to anyone of the preceding claims, wherein said immunotherapy treatment is chosen among anti-PDL1 monotherapy, atezolizumab and bevacizumab combination therapy, anti-PDL1 and anti-VEGF combination therapy, anti-PD1 and CTLA4 combination therapy, PDL1 and CTLA4 combination therapy, anti-VEGFR2 therapy such as ramucirumab therapy, and anti-PD1 therapy such as pembrolizumab therapy.

9. An ex *vivo* method according to anyone of the preceding claims, wherein said immunotherapy treatment is as defined in claim 8, and said proteins involved in the energy metabolism are at least one among mitochondrial proteins, proteins involved in tricarboxylic acid cycle, oxidative metabolism, and fatty acid beta oxidation pathways.

10. An ex *vivo* method according to anyone of the preceding claims, comprising measuring proteins involved in the glycolysis.

11. An ex *vivo* method according to anyone of the preceding claims, wherein said hepatocellular carcinoma is resectable or non resectable hepatocellular carcinoma.

12. An ex *vivo* method according to anyone of the preceding claims, wherein samples are obtainable by at least one technique selected among macrodissection, laser microdissection of a formalin-fixed paraffin-embedded or frozen tissue, fresh biopsy and positon emission tomography.
